# EUROPEAN PATENT APPLICATION

(11) **EP 4 844 054 A1**
(43) Date of publication of application: **25.11.2026**
(21) Application number: 24926958.0
(22) Date of filing: 23.12.2024
(51) Int. Cl.: C12M 1/34

(54) **CELL OBSERVATION DEVICE, METHOD FOR OPERATING CELL OBSERVATION DEVICE, AND PROGRAM FOR OPERATING CELL OBSERVATION DEVICE**

(30) Priority: 26.02.2024 JP 2024026482
(71) Applicant: FUJIFILM Corporation, Tokyo 106-8620 (JP)
(72) Inventor: MATSUBARA, Kenta, Ashigarakami-gun, Kanagawa 258-8577 (JP)
(74) Representative: Hoffmann Eitle
(86) International application number: PCT/JP2024/045506
(87) International publication number: WO 2025/182273

(57) **Abstract**

A cell observation apparatus includes an imaging device configured to capture an image of cells included in a cell suspension flowing through a flow channel, a density detection mechanism configured to detect a density of the cells in the cell suspension, a dilution mechanism configured to dilute the cell suspension in the flow channel, and a processor configured to control the density of the cells by diluting the cell suspension to achieve a target density preset as a density suitable for the imaging device to capture the image.

## Description

### BACKGROUND OF THE INVENTION

### 1. Field of the Invention

The technology of the present disclosure relates to a cell observation apparatus, an operation method for the cell observation apparatus, and an operation program for the cell observation apparatus.

### 2. Description of the Related Art

A cell culture system for culturing cells using a culture vessel is known (see, for example, WO2015/069616A). The culture vessel accommodates a cell suspension in which the cells are suspended in a culture medium. Cell culture requires appropriate determination of a process condition of the culturing process, such as temperature control of the culture vessel, concentration control of the cell suspension in the culture vessel, and control of components in the culture medium, such as vitamins and amino acids.

### SUMMARY OF THE INVENTION

To appropriately determine the process condition, it is necessary to ascertain cell quality, which is the state of cells being cultured, and take the ascertained cell quality into consideration. To this end, cells being cultured are extracted from the culture vessel, and the extracted cells are transferred to a vessel such as a petri dish and observed to ascertain the cell quality. However, such an offline inspection is labor-intensive and time-consuming. Therefore, an inline inspection technique is being studied in which cells being cultured are observed inline while flowing through a flow channel. As a cell observation apparatus, a phase-contrast microscope, three-dimensional image measurement using digital holography technology, and the like are used.

However, in the case of in-line cell observation, the density of cells flowing in the flow channel changes. In such a case, if the cells are imaged in a high-density state, the image quality may be degraded. This is because, given the same amount of illumination light used for imaging, as the cell density increases, insufficiency in exposure amount increases. Therefore, if the cell density is excessively high, an appropriate exposure amount cannot be obtained, which may result in a decrease in contrast. Low-contrast cell images make it difficult to accurately ascertain cell quality, which may hinder the determination of appropriate process management conditions.

The technology of the present disclosure provides a cell observation apparatus, an operation method for the cell observation apparatus, and an operation program for the cell observation apparatus, each capable of acquiring a cell image having improved image quality compared to the related art.

To achieve the above object, a cell observation apparatus according to the technology of the present disclosure includes an imaging device configured to capture an image of cells included in a cell suspension flowing through a flow channel, a density detection mechanism configured to detect a density of the cells in the cell suspension, a dilution mechanism configured to dilute the cell suspension in the flow channel, and a processor configured to control the density of the cells by diluting the cell suspension to achieve a target density preset as a density suitable for the imaging device to capture the image.

Preferably, the density detection mechanism is configured to analyze the image obtained through the imaging device to detect the density.

Preferably, the imaging device is an image measurement device using digital holography technology.

Preferably, the density detection mechanism is a mechanism separate from the imaging device and is configured to detect the density in a section on an upstream side of the imaging device in the flow channel.

Preferably, the processor further has a function of determining a process condition of a culturing process for the cells, based on the image.

Preferably, the flow channel is a flow channel through which the cell suspension that is extracted from a culture vessel for culturing the cells and that includes a cell being cultured flows.

An operation method for a cell observation apparatus according to the technology of the present disclosure is an operation method for a cell observation apparatus including an imaging device configured to capture an image of cells included in a cell suspension flowing through a flow channel, the operation method including detecting a cell density in the cell suspension; and diluting the cell suspension to control the cell density to achieve a target density preset as a density suitable for the imaging device to capture the image.

An operation program for a cell observation apparatus according to the technology of the present disclosure is an operation program for a cell observation apparatus including a processor and an imaging device configured to capture an image of cells included in a cell suspension flowing through a flow channel, the operation program causing the processor to execute a process including detecting a cell density in the cell suspension; and diluting the cell suspension to control the cell density to achieve a target density preset as a density suitable for the imaging device to capture the image.

The technology of the present disclosure enables acquisition of a cell image having improved image quality compared to the related art.

### BRIEF DESCRIPTION OF THE DRAWINGS

Fig. 1 is a diagram illustrating an overview of a cell culture system with a cell observation apparatus incorporated therein;
Fig. 2 is a block diagram illustrating a hardware configuration of the cell observation apparatus;
Fig. 3 is a diagram illustrating an imaging unit using digital holography technology;
Fig. 4 is a diagram conceptually illustrating a principle of generation of interference fringes and an interference fringe image;
Fig. 5 is a diagram illustrating a positional relationship between a flow channel member and the imaging unit;
Fig. 6 is a diagram illustrating dilution control;
Fig. 7 is a diagram illustrating a state of a target density;
Fig. 8 is a diagram illustrating a state in which the density is higher than in Fig. 7;
Fig. 9 is a diagram illustrating a state in which the density is higher than in Fig. 8;
Fig. 10 is a diagram illustrating a relationship between cell density and exposure amount;
Fig. 11 is a diagram illustrating an example of control by a dilution control unit;
Fig. 12 is a flowchart illustrating an operation procedure of the cell observation apparatus;
Fig. 13 is a diagram illustrating a cell observation apparatus according to a second embodiment; and
Fig. 14 is a diagram illustrating a cell observation apparatus having a function of determining a process control condition.

### DESCRIPTION OF THE PREFERRED EMBODIMENTS

### First Embodiment

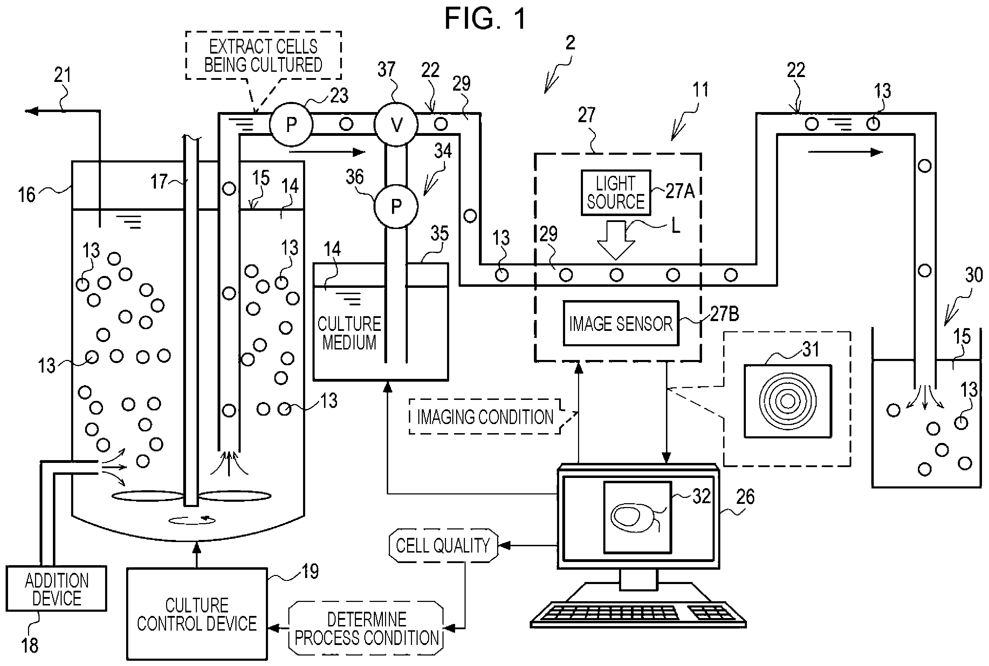

In Fig. 1, a cell observation apparatus 11 is incorporated in a cell culture system 2. The cell culture system 2 includes a culture vessel 16 for culturing cells 13 therein. The culture vessel 16 accommodates a cell suspension 15 obtained by suspending the cells 13 in a culture medium 14. In the cell culture system 2, the culturing process for the cells 13 is appropriately managed.

To appropriately manage the culturing process, for example, a process condition, such as a culture condition, needs to be set to an appropriate condition. Examples of the culture condition include environmental conditions of the culture vessel 16, component replenishment conditions of the culture medium 14, and a replacement timing of the culture medium 14. The environmental conditions of the culture vessel 16 are the temperature of the culture vessel 16, the stirring speed of the cell suspension 15 by a stirring device 17, and the like. The component replenishment conditions of the culture medium 14 are an amount, a timing, and the like of replenishment of the components included in the culture medium 14 by an addition device 18. The components of the culture medium 14 include a carbon source, vitamins, amino acids, and the like. As management of the culturing process, examples of state management of the cell suspension 15 include removal of debris (such as dead cells and residues) in the cell suspension 15 and replacement of the culture medium 14. A culture control device 19 executes such control of the culturing process.

Cultured cells 13 or a product produced by the cells 13 is recovered from the culture vessel 16 through a culture product recovery path 21 from the culture vessel 16 toward a subsequent step. Examples of the process condition also include a recovery timing of the cultured cells 13.

In addition to the culture product recovery path 21, an observation path 22 is provided for the culture vessel 16. The observation path 22 is provided with a pump 23, and the pump 23 extracts the cell suspension 15 including the cells 13 being cultured from the culture vessel 16. The cells 13 being cultured, which flow through a flow channel 29 of the observation path 22, are observed by the cell observation apparatus 11.

The cell observation apparatus 11 is used to ascertain the cell quality of the cells 13 being cultured in order to appropriately manage the culturing process. That is, the cell observation apparatus 11 performs inline inspection of the cells 13 flowing through the flow channel 29 of the observation path 22. Then, the necessity to change the process condition including the culture condition and the content of the change are determined based on the cell quality of the cells 13 being cultured. Furthermore, based on the cell quality, operations such as determining a timing for removing debris, a timing for replacing the culture medium 14, and a timing for recovering a culture product are performed.

The cell observation apparatus 11 includes a control device 26 and an imaging unit 27. The imaging unit 27 is disposed in the observation path 22. The cells 13 that flow through the observation path 22 and are imaged are stored in a storage portion 30. For example, some of the cells 13 in the storage portion 30 are returned to the culture vessel 16 and others are discarded according to the cell state.

The imaging unit 27 has a light source 27A that emits illumination light L for illuminating the cells 13 to be observed, and an image sensor 27B that captures an optical image of the illuminated light source 27A. As an example, the cell observation apparatus 11 is an image measurement device using digital holography technology. The cell observation apparatus 11 measures, for example, an image representing the three-dimensional shape of the cells 13. The image representing the three-dimensional shape of the cells 13 is also referred to as a three-dimensional image. Observing the three-dimensional shape of the cells 13 enables more accurate ascertainment of the cell quality. The imaging unit 27 is an example of an "imaging device" according to the technology of the present disclosure.

The image sensor 27B captures, as an optical image, an interference fringe image 31 representing an interference state between object light and reference light. The control device 26 controls driving of the imaging unit 27 and executes image processing for reconstructing a cell image 32 representing the three-dimensional shape of the cells 13, based on the interference fringe image 31.

The cell quality of the cells 13 being cultured is ascertained based on the cell image 32 acquired by the cell observation apparatus 11, and the process condition including the culture condition of the culturing process described above is determined based on the ascertained cell quality. The culturing process is controlled by the culture control device 19 in accordance with the determined process condition.

The cell observation apparatus 11 further includes a dilution mechanism 34 that dilutes the cell suspension 15 in the flow channel 29 of the observation path 22. The dilution mechanism 34 includes a culture medium tank 35, a pump 36, and a valve 37. The culture medium tank 35 accommodates the culture medium 14 for dilution. The pump 36 draws the culture medium 14 from the culture medium tank 35 into the observation path 22. The valve 37 opens and closes an inlet through which the culture medium 14 from the culture medium tank 35 flows into the observation path 22. The culture medium 14 drawn from the culture medium tank 35 is added to the observation path 22 to dilute the cell suspension 15, thereby enabling a reduction in the density of the cells 13.

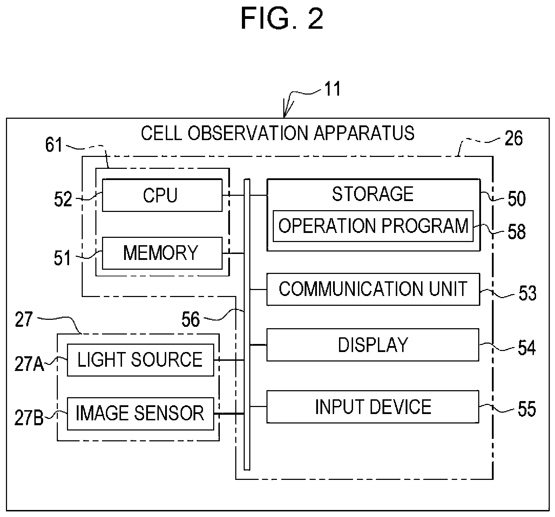

As illustrated in Fig. 2, in the cell observation apparatus 11, the control device 26 is constituted by, for example, a computer. The control device 26 includes a storage 50, a memory 51, a central processing unit (CPU) 52, a communication unit 53, a display 54, and an input device 55. These are interconnected via a busline 56.

The storage 50 is a hard disk drive installed in a computer constituting the control device 26 or connected via a cable or a network. Alternatively, the storage 50 is a disk array in which a plurality of hard disk drives are arranged. The storage 50 stores a control program such as an operating system, various application programs, various data associated with these programs, and the like. A solid-state drive may be used instead of the hard disk drive.

The memory 51 is a work memory for the CPU 52 to execute processing. The CPU 52 loads a program stored in the storage 50 into the memory 51 and executes processing according to the program, thereby integrally controlling each part of the computer.

The storage 50 further stores an operation program 58 according to the technology of the present disclosure. The operation program 58 is an application program for causing the computer to function as the cell observation apparatus 11. The CPU 52 executes the operation program 58, thereby functioning as a processor 61 according to the technology of the present disclosure in cooperation with the memory 51.

The communication unit 53 is a network interface that performs transmission control of various kinds of information via a network such as a local area network (LAN). The display 54 displays various screens. The computer constituting the control device 26 receives input of operation instructions from the input device 55 through the various screens. The input device 55 is a keyboard, a mouse, a touch panel, or the like.

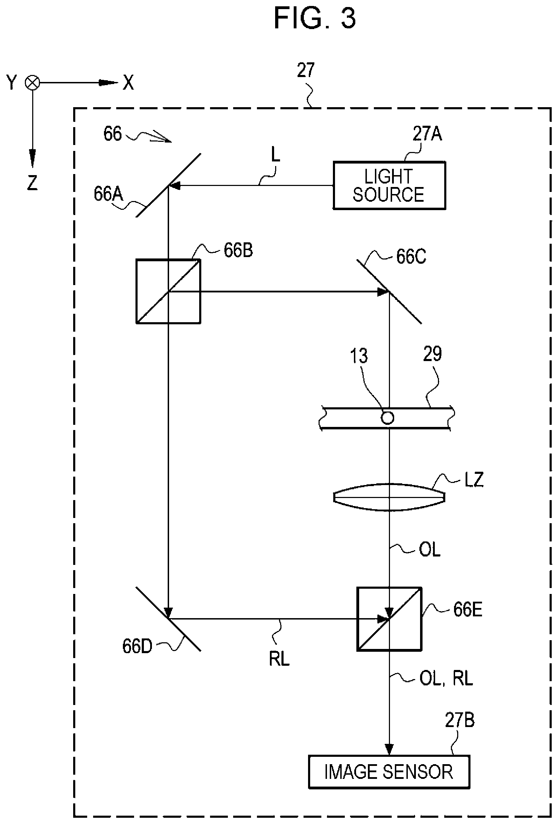

As illustrated in Fig. 3, the imaging unit 27 includes an imaging optical system 66 in addition to the light source 27A and the image sensor 27B. As is well known, digital holography is a technique for recording phase information of light in addition to intensity information of light of an object to be observed by using coherence of light, and for reproducing a three-dimensional image of the object by using the recorded intensity information and phase information.

In digital holography, coherent laser light is used as the illumination light L in order to utilize interference of light. The light source 27A is a laser light source that emits laser light as the illumination light L. The image sensor 27B is a two-dimensional image sensor having an image pick-up surface provided with a two-dimensional array of light-receiving elements. For example, a complementary metal-oxide semiconductor (CMOS) image sensor or the like is used.

The imaging optical system 66 is disposed in an optical path extending from the light source 27A to the image sensor 27B. As an example, the imaging optical system 66 includes a first reflection mirror 66A, a beam splitter 66B, a second reflection mirror 66C, an imaging lens LZ, a third reflection mirror 66D, and a beam combiner 66E. The first reflection mirror 66A reflects the illumination light L emitted from the light source 27A toward the beam splitter 66B. The beam splitter 66B separates the illumination light L into an object light path directed toward the second reflection mirror 66C and a reference light path directed toward the third reflection mirror 66D.

In the object light path including the second reflection mirror 66C, the flow channel 29 through which the cells 13 pass is disposed, and the cells 13 are irradiated with the illumination light L reflected by the second reflection mirror 66C. The illumination light L that has passed through the flow channel 29 and the cells 13 is incident on the imaging lens LZ as object light OL. The imaging lens LZ condenses the object light OL and forms an optical image of the condensed object light OL on the image pick-up surface of the image sensor 27B through the beam combiner 66E.

On the other hand, the illumination light L traveling through the reference light path from the beam splitter 66B toward the third reflection mirror 66D is incident on the third reflection mirror 66D without passing through the flow channel 29 through which the cells 13 flow. The illumination light L reflected by the third reflection mirror 66D is incident on the beam combiner 66E as reference light RL. Then, the reference light RL is incident on the image sensor 27B through the beam combiner 66E.

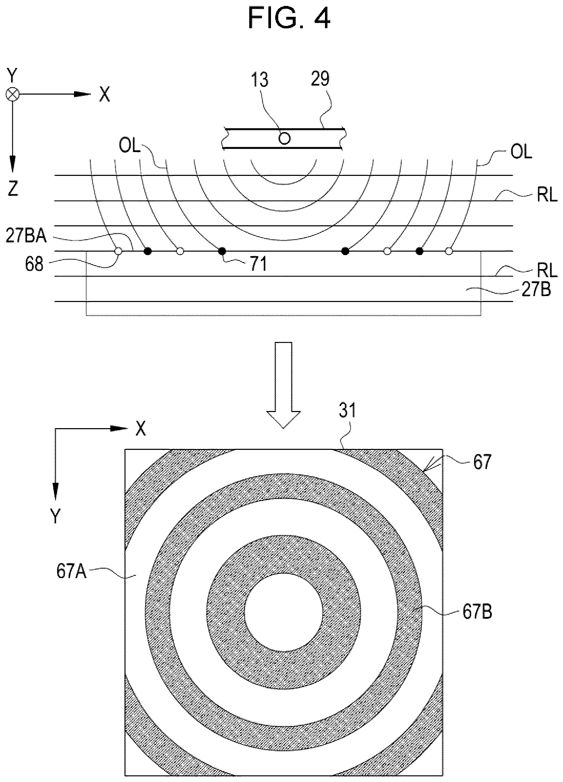

As conceptually illustrated in Fig. 4, the object light OL transmitted through the flow channel 29 and the cells 13 is diffracted by the cells 13 and substances constituting the flow channel 29, resulting in a curved wavefront. In contrast, the reference light RL not passing through the flow channel 29 and the cells 13 is not diffracted by the cells 13 and the like, resulting in a plane wave. The object light OL has a phase difference relative to the reference light RL, and the phase difference has a magnitude according to the refractive indices of the cells 13, the substances constituting the flow channel 29, and the like. The object light OL and the reference light RL interfere with each other on an image pick-up surface 27BA of the image sensor 27B, thereby generating interference fringes 67. The image sensor 27B captures an image of the interference fringes 67 and outputs the interference fringe image 31.

The pixel values of the respective pixels in the interference fringe image 31 indicate the intensities of the interference fringes 67. That is, the interference fringe image 31 is a two-dimensional distribution of the intensities of the interference fringes 67. The intensities of the interference fringes 67 vary in the image pick-up surface 27BA (X-Y plane) in accordance with the phase difference between the object light OL and the reference light RL.

Among lines representing the object light OL and the reference light RL, solid lines indicate wavefronts of the object light OL and the reference light RL at the maximum amplitude. In contrast, broken lines indicate wavefronts of the object light OL and the reference light RL at the minimum amplitude. White points 68 illustrated on the image pick-up surface 27BA are portions where wavefronts of the object light OL and the reference light RL are aligned to reinforce each other. The portions corresponding to the white points 68 appear as bright portions 67A in the interference fringes 67. In contrast, black points 71 illustrated on the image pick-up surface 27BA are portions where wavefronts of the object light OL and the reference light RL are shifted by a half wavelength to weaken each other. The portions corresponding to the black points 71 appear as dark portions 67B in the interference fringes 67.

For example, depending on an image reconstruction method for digital holography, a plurality of interference fringe images 31 captured while varying an optical path difference between the object light OL and the reference light RL are used. Also regarding the illumination light L, interference fringe images 31 captured using, for example, three or more illumination lights L having different wavelengths are acquired. Based on the plurality of interference fringe images 31, image reconstruction of the cell image 32 representing the three-dimensional shape of the cells 13 is performed.

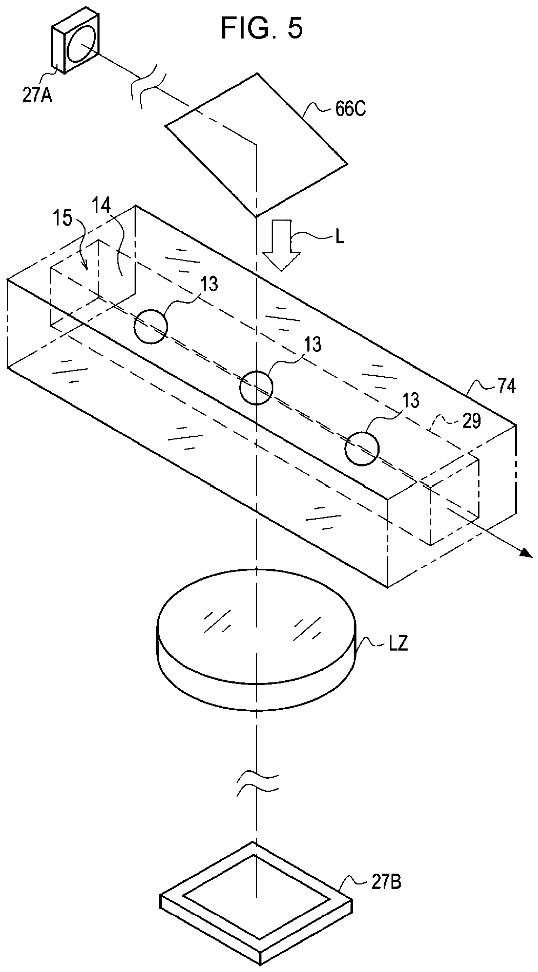

As illustrated in Fig. 5, the flow channel 29 is formed in, for example, a flow channel member 74. The flow channel member 74 is a plate-shaped member transparent to the illumination light L, and is made of, for example, plastic. The flow channel 29 is defined by, for example, a groove formed in part of the flow channel member 74, and the formed groove is sealed with a cover. The flow channel member 74 is also referred to as a microchannel device or the like.

The illumination light L is transmitted through the flow channel member 74 and the cells 13. Since the cell suspension 15 including the cells 13 flows through the flow channel 29, the illumination light L is also transmitted through the culture medium 14 other than the cells 13 in the cell suspension 15. In the example illustrated in Fig. 5, a cross-sectional shape of the flow channel 29 orthogonal to a flow direction of the flow channel 29 is rectangular. It is to be understood that the cross-sectional shape of the flow channel 29 may be circular. The flow channel member 74 may be a tube or the like instead of a plate-shaped member.

As an example, the imaging unit 27 repeatedly performs still-image capture of the interference fringe image 31 at preset intervals. Accordingly, interference fringe images 31 in which the cells 13 flowing through the flow channel 29 appear are captured. It is to be understood that the interference fringe images 31 may be recorded as a moving image.

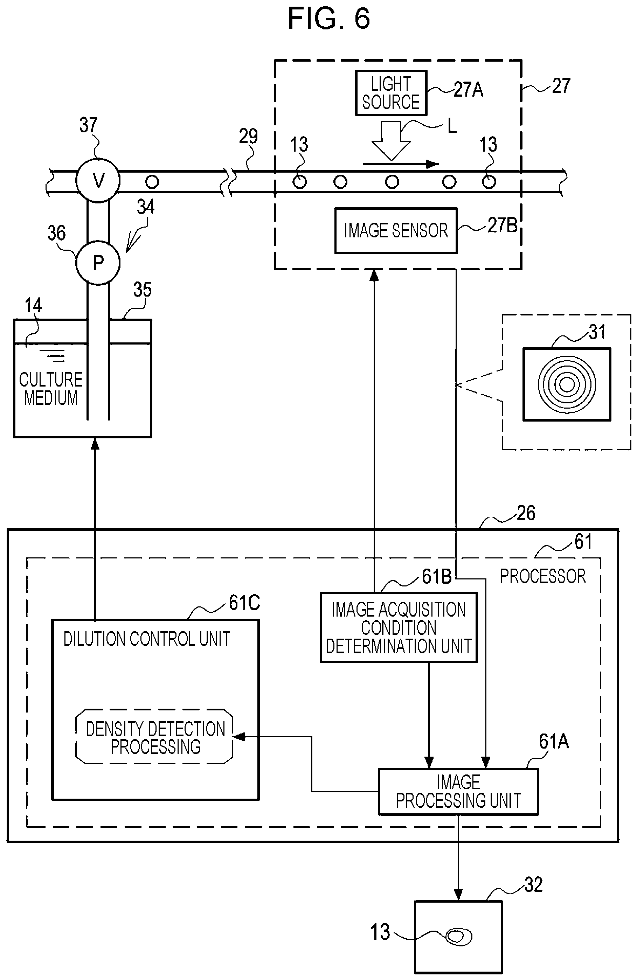

As illustrated in Fig. 6, the processor 61 of the control device 26 functions as an image processing unit 61A, an image acquisition condition determination unit 61B, and a dilution control unit 61C. The image processing unit 61A acquires an interference fringe image 31 from the imaging unit 27 and reconstructs a cell image 32 based on the acquired interference fringe image 31. The image acquisition condition determination unit 61B determines an image acquisition condition including an imaging condition of the imaging unit 27 and an image processing condition of the image processing unit 61A. The imaging condition includes an exposure condition such as the amount of the illumination light L.

The dilution control unit 61C analyzes the cell image 32 reconstructed by the image processing unit 61A to detect a cell density in the cell suspension 15 in the flow channel 29. That is, the dilution control unit 61C, the imaging unit 27, and the image processing unit 61A constitute a "density detection mechanism" according to the technology of the present disclosure.

In the processor 61, the dilution control unit 61C causes the dilution mechanism 34 to dilute the cell suspension 15 in the flow channel 29 to control the density of the cells 13. Specifically, the dilution control unit 61C compares a measured value of the density detected by the density detection mechanism with a target density preset as a density suitable for imaging by the imaging unit 27. In a case where the measured value exceeds the target density, the dilution control unit 61C dilutes the cell suspension 15 to control the density of the cells 13 to achieve the target density.

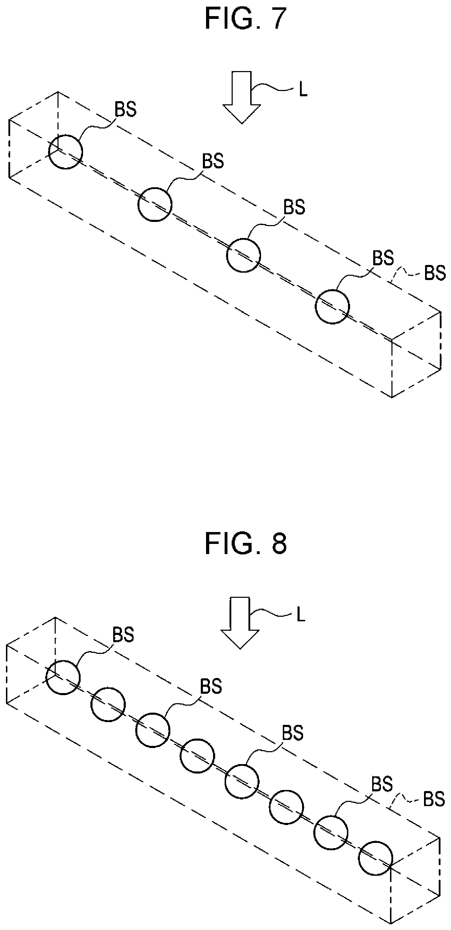

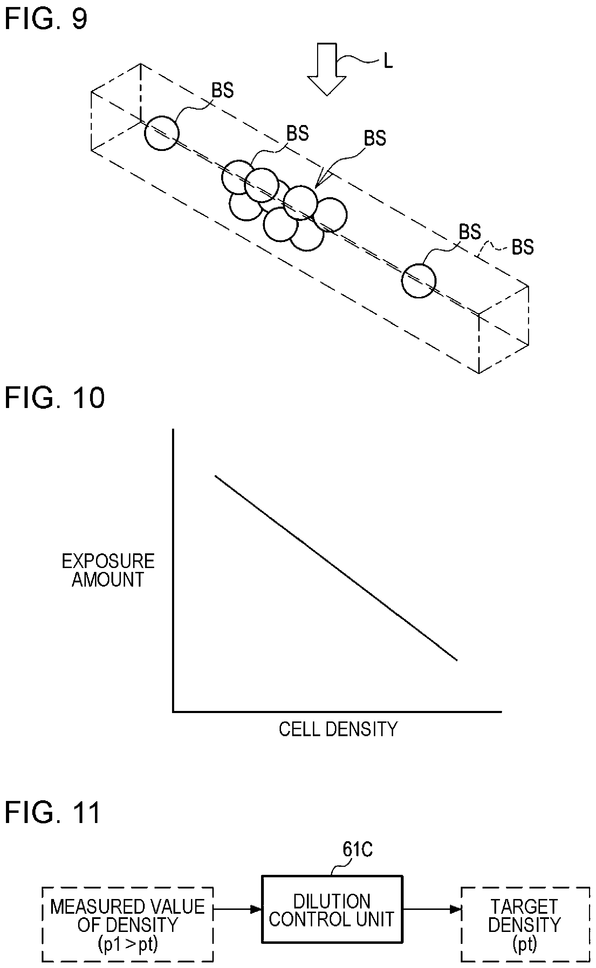

The density of the cells 13 is controlled to improve the image quality of the cell image 32. That is, in the imaging unit 27, if the exposure amount of the image sensor 27B is excessively low, the contrast of the cell image 32 decreases. States illustrated in Figs. 7 to 9 in which the density of the cells 13 differs are compared given the same amount of the illumination light L. Fig. 7 illustrates a state in which the density of the cells 13 is the target density. In this case, the exposure amount of the image sensor 27B is appropriate. Consequently, the cell image 32 having good contrast is obtained. In the state illustrated in Fig. 8 in which the density is higher than that in Fig. 7, in contrast, the amount by which the illumination light L is blocked by the cells 13 in the flow channel 29 is larger. Thus, the exposure amount of the image sensor 27B is lower than that in the state illustrated in Fig. 7. In the state illustrated in Fig. 9, a plurality of cells 13 overlap and aggregate in the flow channel 29, thereby forming a cell aggregate 13S. In the state illustrated in Fig. 9, the amount by which the illumination light L is blocked further increases compared to the state illustrated in Fig. 8, and the exposure amount of the image sensor 27B decreases compared to the state illustrated in Fig. 8. That is, the density of the cells 13 and the exposure amount have a relationship as illustrated in Fig. 10.

Therefore, the dilution control unit 61C controls the density of the cells 13 in the cell suspension 15 in the flow channel 29 to achieve the target density through the dilution mechanism 34. For example, as illustrated as an example in Fig. 11, in a case where the measured value of the density in the flow channel 29 is denoted by ρ1 and exceeds a target density ρt, the dilution control unit 61C causes the dilution mechanism 34 to add the culture medium 14 to the flow channel 29. Accordingly, the density in the flow channel 29 is controlled to achieve the target density ρt. The target density ρt is a value preset as a density suitable for the imaging unit 27 to capture an image. Specifically, a density is ideally such that the cells 13 appear one by one in one interference fringe image 31.

Maintaining the density in the flow channel 29 at the target density ρt suppresses a decrease in contrast in the cell image 32. Since the image quality of the cell image 32 is improved, it is possible to accurately ascertain cell quality.

As illustrated in Figs. 8 and 9, if the density of the cells 13 is excessively high, the number of cells 13 appearing in the interference fringe image 31 is excessively large, or a plurality of cells 13 appear overlapping each other. In such a case, the image quality of the cell image 32 degrades in the sense that it is difficult to clearly ascertain the state of each individual cell 13. Controlling the density of the cells 13 to achieve the target density also suppresses such a degradation in image quality.

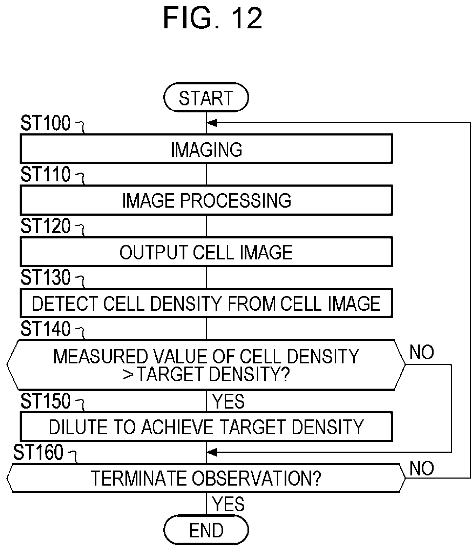

Operations of the above configuration will be described with reference to a flowchart illustrated in Fig. 12. In the cell culture system 2, after culturing is started, the cell suspension 15 including the cells 13 is extracted from the culture vessel 16 during culturing. The extracted cell suspension 15 flows through the flow channel 29 and is imaged by the imaging unit 27 in step ST100. The processor 61 acquires an interference fringe image 31 of an imaged cell 13. In step ST110, the processor 61 performs image reconstruction processing based on the acquired interference fringe image 31 to reconstruct a cell image 32. In step ST120, the processor 61 outputs the cell image 32 to the display 54.

The operator checks the cell 13 appearing in the displayed cell image 32 and ascertains the cell quality. Then, the operator determines a process condition of the culturing process in accordance with the cell quality, and sets the determined process condition in the culture control device 19. Accordingly, the process condition is set and changed according to the cell quality. As a result, appropriate cell culture is performed. Furthermore, the frequency of monitoring for checking the cell quality may be changed in accordance with the cell quality. For example, the number of checks of the cell quality may be increased in a case where the cell quality has not reached a target.

In step ST130, the processor 61 of the cell observation apparatus 11 analyzes the cell image 32 to detect the density of the cells 13 in the cell suspension 15 flowing through the flow channel 29. Then, in step ST140, the processor 61 determines whether the measured value of the density of the cells 13 exceeds the target density. In a case where the measured value is less than or equal to the target density (NO in step ST140), the process proceeds to step ST160. In a case where the measured value exceeds the target density (YES in step ST140), the processor 61 dilutes the density of the cells 13 to the target density through the dilution mechanism 34. Accordingly, the density of the cells 13 is maintained at the target density. In step ST160, the processor 61 determines whether the observation is to be terminated, and repeatedly performs the above processing until the observation ends.

The cell observation apparatus 11 according to the technology of the present disclosure includes the imaging unit 27 (an example of an imaging device) configured to capture an image (the interference fringe image 31 as an example) of the cells 13 included in the cell suspension 15 flowing through the flow channel 29, a density detection mechanism (the processor 61 as an example) configured to detect the density of the cells 13 in the cell suspension 15, the dilution mechanism 34 configured to dilute the cell suspension 15 in the flow channel 29, and the processor 61 configured to control the density of the cells 13 by diluting the cell suspension 15 to achieve a target density preset as a density suitable for the imaging unit 27 to capture the image. This allows the exposure amount of the imaging unit 27 to be appropriately controlled through the control of the density of the cells 13, thereby suppressing contrast degradation in the cell image 32. Consequently, the cell observation apparatus 11 can acquire the cell image 32 having improved image quality compared to the related art. The cell image 32 having improved image quality enables accurate ascertainment of the cell quality, thereby allowing an appropriate process condition to be determined.

In the above embodiment, the density detection mechanism analyzes the cell image 32 obtained through the imaging unit 27 to detect the density. Therefore, a dedicated density detection mechanism separate from the imaging unit 27 is unnecessary.

In the above embodiment, furthermore, the cell observation apparatus 11 includes an imaging device (as an example, the imaging unit 27) that captures an image of a cell 13, and the imaging device is an image measurement device using digital holography technology. In a case where an image measurement device using digital holography technology is used, the density of the cells 13 may have a relatively large influence on image quality. Therefore, the technology of the present disclosure is very effective.

The imaging unit 27 is disposed in the observation path 22. The flow channel 29 of the observation path 22 is a flow channel through which the cell suspension 15 extracted from the culture vessel 16 for culturing the cells 13 and including the cells 13 being cultured flows. Therefore, the state of the cells being cultured can be ascertained, and a process condition of the culturing process can be controlled during cell culture.

Since the flow channel 29 of the observation path 22 is a flow channel for observing the cells 13 being cultured, the range of changes in the cell quality of the cells 13 is also broad, unlike the flow channel of the culture product recovery path 21 through which the cultured cells 13 flow. Therefore, in a case where the cells 13 being cultured are observed, the necessity to accurately ascertain cell quality is higher than in a case where the cultured cells 13 are observed, and the technology of the present disclosure is particularly effective.

### Second Embodiment

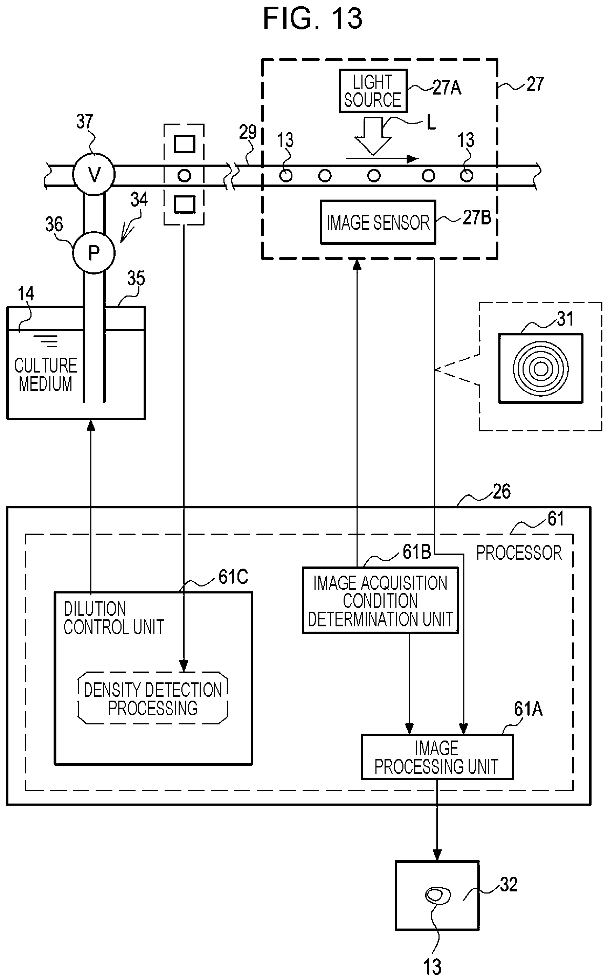

Unlike the first embodiment, a cell observation apparatus 11 according to a second embodiment illustrated in Fig. 13 includes a photosensor 76 disposed on the upstream side of the imaging unit 27 in the observation path 22. Other configurations are similar to those of the first embodiment except that processing using the photosensor 76 is different.

The photosensor 76 and the processor 61 constitute a density detection mechanism separate from the imaging unit 27. As an example, the photosensor 76 is a transmission-type photosensor in which a light-emitting element 76A and a light-receiving element 76B are disposed facing each other with the flow channel 29 of the observation path 22 interposed therebetween. In the photosensor 76, as is well known, the light-receiving element 76B receives detection light emitted from the light-emitting element 76A. Upon an object such as a cell 13 passing between the light-emitting element 76A and the light-receiving element 76B, part of the detection light is blocked by the object, thereby changing the amount of light received by the light-receiving element 76B. The light-receiving element 76B outputs a detection signal corresponding to the amount of received light to the processor 61. Instead of the transmission-type photosensor, a reflection-type photosensor may be used as the photosensor 76.

The processor 61 can detect the cells 13 passing through the photosensor 76, based on a change in the detection signal received from the photosensor 76 and corresponding to the amount of received light. By measuring an average period of the detection signal that periodically changes in a case where a plurality of cells 13 pass through the photosensor 76, the processor 61 can detect a density of a plurality of cells 13 passing within a certain period of time. Furthermore, by measuring a degree to which the detection signal drops and a time during which the detection signal remains dropped, the processor 61 can detect a size of the cell aggregate 13S. This is because a decrease in the amount of light received by the light-receiving element 76B per unit time is larger and the duration of the decrease is longer in a case where the cell aggregate 13S passes than in a case where a single cell 13 passes. For example, as the size of the cell aggregate 13S increases, the density of the cells 13 can be evaluated to increase.

As described above, the density detection mechanism constituted by the photosensor 76 and the processor 61 detects the density of the cells 13 in a section on the upstream side of the imaging unit 27 in the flow channel 29. Such a density detection mechanism can simplify density detection processing compared to a case where the density of the cells 13 is detected through image analysis of the cell image 32.

### Modifications

In the above embodiments, an operator ascertains the cell quality based on the cell image 32 output from the cell observation apparatus 11, and determines a process condition of the culturing process based on the ascertained cell quality. Such a function may be added to the cell observation apparatus 11.

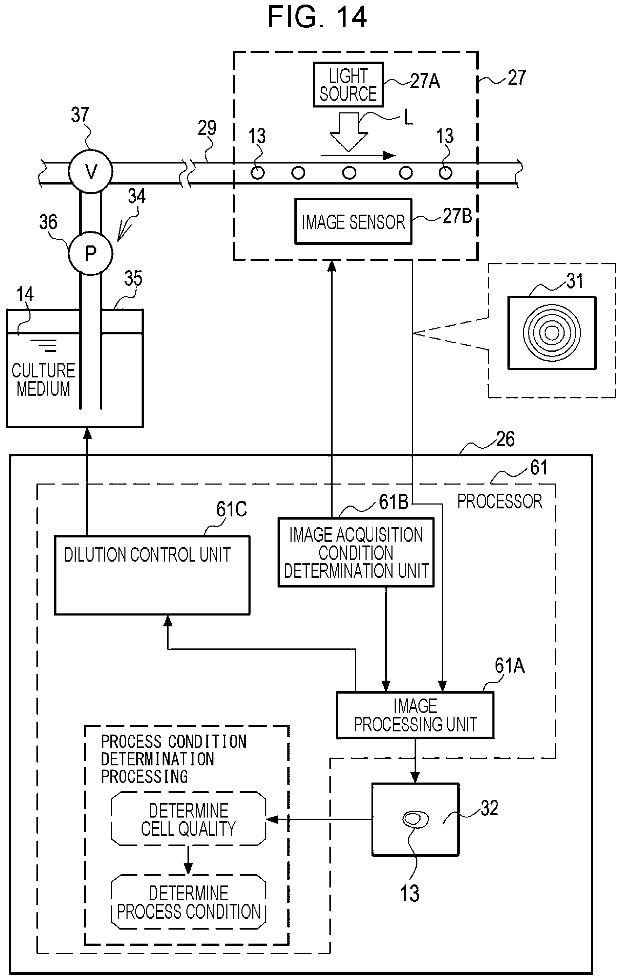

In the cell observation apparatus 11 illustrated in Fig. 14, the processor 61 has a function of determining a process condition of the culturing process for the cells 13 based on the cell image 32. The processor 61 executes process condition determination processing. In the process condition determination processing, the processor 61 performs image analysis on the cell image 32 to determine the cell quality of the cells 13. Based on the determination result, the processor 61 determines a process condition including the above-described culture condition for the cells 13.

While an image measurement device using digital holography technology has been described as an example of the cell observation apparatus 11, a phase-contrast microscope, a brightfield microscope, or the like may also be used as the cell observation apparatus 11. For example, exposure condition control based on the flow channel information is also effective for a phase-contrast microscope and a brightfield microscope.

Various modifications may be made to the hardware configuration of a computer constituting the cell observation apparatus 11. For example, for improvements in processing capability and reliability, the cell observation apparatus 11 may also be constituted by a plurality of computers that are separate in terms of hardware.

As described above, the hardware configuration of the computer of the cell observation apparatus 11 may be changed as appropriate in accordance with performance requirements such as processing capability, safety, and reliability. Furthermore, it is to be understood that, in addition to the hardware, application programs such as the operation program 58 may also be duplicated or stored in a plurality of storage devices in a distributed manner for ensuring safety and reliability.

The above description enables understanding of the technology defined in the following appendices.

### Appendix 1

A cell observation apparatus including:
an imaging device configured to capture an image of cells included in a cell suspension flowing through a flow channel;
a density detection mechanism configured to detect a density of the cells in the cell suspension;
a dilution mechanism configured to dilute the cell suspension in the flow channel; and
a processor configured to control the density of the cells by diluting the cell suspension to achieve a target density preset as a density suitable for the imaging device to capture the image.

### Appendix 2

The cell observation apparatus according to Appendix 1, wherein
the density detection mechanism is configured to analyze the image obtained through the imaging device to detect the density.

### Appendix 3

The cell observation apparatus according to Appendix 1 or Appendix 2, wherein
the imaging device is an image measurement device using digital holography technology.

### Appendix 4

The cell observation apparatus according to any one of Appendices 1 to 3, wherein
the density detection mechanism is a mechanism separate from the imaging device and is configured to detect the density in a section on an upstream side of the imaging device in the flow channel.

### Appendix 5

The cell observation apparatus according to any one of Appendices 1 to 4, wherein
the processor further has a function of determining a process condition of a culturing process for the cells, based on the image.

Appendix 6 The cell observation apparatus according to any one of Appendices 1 to 5, wherein
the flow channel is a flow channel through which the cell suspension that is extracted from a culture vessel for culturing the cells and that includes a cell being cultured flows.

### Appendix 7

An operation method for a cell observation apparatus including an imaging device configured to capture an image of cells included in a cell suspension flowing through a flow channel, the operation method including:
detecting a cell density in the cell suspension; and
diluting the cell suspension to control the cell density to achieve a target density preset as a density suitable for the imaging device to capture the image.

### Appendix 8

An operation program for a cell observation apparatus including a processor and an imaging device configured to capture an image of cells included in a cell suspension flowing through a flow channel, the operation program causing the processor to execute a process including:
detecting a cell density in the cell suspension; and
diluting the cell suspension to control the cell density to achieve a target density preset as a density suitable for the imaging device to capture the image.

In the above embodiments, the hardware structures of processing units that execute various processes, such as the image processing unit 61A, the image acquisition condition determination unit 61B, and the dilution control unit 61C, may be implemented using various processors described below. As described above, the various processors include the CPU 52, which is a general-purpose processor configured to execute software (the operation program 58) to function as various processing units, and further include a programmable logic device (PLD) that is a processor whose circuit configuration can be changed after manufacture, such as a field programmable gate array (FPGA), a dedicated electric circuit that is a processor having a circuit configuration designed exclusively for executing specific processing, such as an application specific integrated circuit (ASIC), and so on.

A single processing unit may be constituted by one of the various processors, or may be constituted by a combination of two or more processors of the same type or different types (for example, a combination of a plurality of FPGAs, and/or a combination of a CPU and an FPGA). Alternatively, a plurality of processing units may be configured as a single processor.

Examples of configuring a plurality of processing units with a single processor include, first, a form in which, as typified by computers such as a client and a server, a single processor is constituted by a combination of one or more CPUs and software, and the processor functions as the plurality of processing units. The examples include, second, a form in which, as typified by a system on chip (SoC) or the like, a processor is used in which the functions of the entire system including the plurality of processing units are implemented as one integrated circuit (IC) chip. As described above, the various processing units are configured by using one or more of the various processors described above as a hardware structure.

More specifically, the hardware structure of these various processors may be an electric circuit (circuitry) in which circuit elements such as semiconductor elements are combined.

In the technology of the present disclosure, the various embodiments and various modifications described above may be combined as appropriate. It is to be understood that the technology of the present disclosure is not limited to the above embodiments, and that various configurations may be adopted without departing from the gist thereof. Furthermore, the technology of the present disclosure encompasses not only a program but also a storage medium storing the program in a non-transitory manner. Examples of the storage medium include a non-transitory computer-readable storage medium such as a universal serial bus (USB) memory, a flexible disk, and a compact disc read only memory (CD-ROM). The program may be provided online via a network such as the Internet. The technology of the present disclosure encompasses not only the program but also a program product. The program product includes any form of product for providing the program. Similarly to the program, the program product may be provided by being stored in a non-transitory computer-readable storage medium, or may be provided online.

The description and drawings presented above provide detailed descriptions of portions according to the technology of the present disclosure and are merely examples of the technology of the present disclosure. For example, the descriptions related to the configurations, functions, operations, and effects described above are descriptions related to an example of the configurations, functions, operations, and effects of portions according to the technology of the present disclosure. Thus, it goes without saying that unnecessary portions may be deleted or new elements may be added or substituted in the description and drawings presented above without departing from the gist of the technology of the present disclosure. To avoid complexity and facilitate understanding of portions according to the technology of the present disclosure, descriptions related to common general technical knowledge and the like, for which no specific explanation is required to implement the technology of the present disclosure, are omitted in the description and drawings presented above.

In this specification, "A and/or B" is synonymous with "at least one of A or B". That is, "A and/or B" means only A, only B, or a combination of A and B. In this specification, furthermore, a concept similar to that of "A and/or B" is applied also to the expression of three or more matters in combination with "and/or".

The disclosure of JP2024-026482 filed on February 26, 2024 is incorporated herein by reference in its entirety. All publications, patent applications, and technical standards mentioned herein are incorporated herein by reference to the same extent as if each individual publication, patent application, and technical standard were specifically and individually indicated to be incorporated by reference.

## Claims

1. A cell observation apparatus comprising:
an imaging device configured to capture an image of cells included in a cell suspension flowing through a flow channel;
a density detection mechanism configured to detect a density of the cells in the cell suspension;
a dilution mechanism configured to dilute the cell suspension in the flow channel; and
a processor configured to control the density of the cells by diluting the cell suspension to achieve a target density preset as a density suitable for the imaging device to capture the image.

2. The cell observation apparatus according to claim 1, wherein
the density detection mechanism is configured to analyze the image obtained through the imaging device to detect the density.

3. The cell observation apparatus according to claim 1, wherein
the imaging device is an image measurement device using digital holography technology.

4. The cell observation apparatus according to claim 1, wherein
the density detection mechanism is a mechanism separate from the imaging device and is configured to detect the density in a section on an upstream side of the imaging device in the flow channel.

5. The cell observation apparatus according to claim 1, wherein
the processor further has a function of determining a process condition of a culturing process for the cells, based on the image.

6. The cell observation apparatus according to claim 1, wherein
the flow channel is a flow channel through which the cell suspension that is extracted from a culture vessel for culturing the cells and that includes a cell being cultured flows.

7. An operation method for a cell observation apparatus including an imaging device configured to capture an image of cells included in a cell suspension flowing through a flow channel, the operation method comprising:
detecting a cell density in the cell suspension; and
diluting the cell suspension to control the cell density to achieve a target density preset as a density suitable for the imaging device to capture the image.

8. An operation program for a cell observation apparatus including a processor and an imaging device configured to capture an image of cells included in a cell suspension flowing through a flow channel, the operation program causing the processor to execute a process comprising:
detecting a cell density in the cell suspension; and
diluting the cell suspension to control the cell density to achieve a target density preset as a density suitable for the imaging device to capture the image.
